(19) Europäisches Patentamt
European Patent Office
Office européen des brevets

(11) Publication number: **0 002 289**
**B1**

(12) # EUROPEAN PATENT SPECIFICATION

(45) Date of publication of patent specification: **09.09.81**

(21) Application number: **78200196.0**

(22) Date of filing: **18.09.78**

(51) Int. Cl.³: **C 07 B 19/00**, C 07 B 20/00, C 07 C 121/75

(54) **Process for converting a stereoisomeric ester into its diastereoisomer; crystalline (-)-S-alpha-cyano-3-phenoxybenzyl (+)-S-alpha-isopropyl-4-chlorophenylacetate.**

(30) Priority: **26.09.77 US 836628**
**30.03.78 US 891773**

(43) Date of publication of application:
**13.06.79 Bulletin 79/12**

(45) Publication of the grant of the European patent:
**09.09.81 Bulletin 81/36**

(84) Designated Contracting States:
**BE CH DE FR GB NL**

(56) References cited:

**DE - A - 2 718 038**
**DE - A - 2 718 039**
**DE - A - 2 727 326**
**FR - A - 2 240 914**

**HOUBEN-WEYL "Methoden der organischen Chemie" Band IV, Teil 2, 1955 Georg Thieme Verlag, Stuttgart, Seiten 511 bis 513**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Soloway, Samuel Barney**
**3401 Mansfield Lane**
**Modesto California 95350 (US)**
Inventor: **Tieman, Charles Henry**
**2209 Fremont Street**
**Modesto California 95350 (US)**

(74) Representative: **Keuzenkamp, Abraham et al,**
**P.O. Box 302**
**NL-2501 CH Den Haag (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

Process for converting a stereoisomeric ester into its diastereoisomer; crystalline (—)-S-alpha-cyano-3-phenoxybenzyl (+)-S-alpha-isopropyl-4-chlorophenylacetate.

This invention relates to a process for the conversion of one stereoisomeric ester into its corresponding diastereoisomer. More particularly it is concerned with the conversion of (+)-R-*alpha*-cyano-phenoxybenzyl (+)-S-*alpha*-isopropyl-4-chlorophenylacetate (Isomer 1) into (—)-S-*alpha*-cyano-3-phenoxybenzyl (+)-S-*alpha*-isopropyl-4-chlorophenylacetate (Isomer 2).

Isomers 1 and 2 are two of the four possible stereoisomers having the following structural formula:

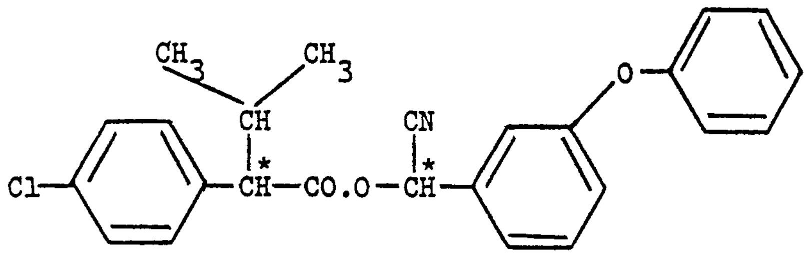

(A)

The asterisks indicate the two asymmetric carbon atoms which give rise to the four possible stereoisomers.

Ester A is described in Belgian Patent 801,946 as having pyrethroid-like properties, including pesticidal activity and low toxicity to mammals. It is further known how to prepare or to recover the (+)-S-form of the acid portion of Ester A, e.g., Japanese Patent Publications 75/25,544 and 75/106,935, and that the pyrethroid esters of such (+)-S-acids are twice as insecticidally-active as the corresponding racemate derived from both the (+)-S- and (—)-R- forms of such acids. The *alpha*-cyano-3-phenoxybenzyl ester diastereoisomer pair derived from the (+)-S- form of *alpha*-isopropyl-4-chlorophenylacetic acid can be separated (resolved) in small quantities into oily liquids by methods such as liquid chromatography.

We have now found an efficient route to convert isomer 1 into isomer 2, or in other words, converting the less insecticidally active isomer into the more insecticidally active isomer.

The invention provides a process for the conversion of (+)-R-*alpha*-cyano-3-phenoxybenzyl (+)-S-*alpha*-isopropyl-4-chlorophenylacetate (hereinafter referred to as isomer 1) into (—)-S-*alpha*-cyano-3-phenoxybenzyl (+)-S-alpha-isopropyl-4-chlorophenylacetate (hereinafter referred to as isomer 2), characterized by treating with a base a solution of isomer 1, alone or in admixture with isomer 2, in a solvent from which isomer 2 crystallises more readily than isomer 1, and removing isomer 2 from the solution in order to permit the base to convert isomer 1 into isomer 2 up to the point at which equimolar proportions of the low isomers are present.

The process according to the invention may be used to convert substantially pure isomer 1 into isomer 2 by treatment with a base followed by isolation of the desired isomer 2 from the resulting mixture of substantially equimolar proportions of isomers 1 and 2.

Also mixtures of isomers 1 and 2 containing a higher proportion of isomer 1 than isomer 2 can be used as starting material in the process according to the invention. Again treatment with the base produces a substantially equimolar mixture of isomers 1 and 2 from which the desired isomer 2 may be separated.

When equimolar proportions of isomers 1 and 2 are used as starting material in the process according to the invention it is necessary to remove a proportion of isomer 2 therefrom in order to permit the base to convert isomer 1 into isomer 2 up to the point at which equimolar proportions of the two isomers are present once again. By removing further isomer 2 from the equimolar mixture the base can then convert more isomer 1 to isomer 2.

Isomers 1 and 2 are esters of an acid containing an asymmetric carbon atom with an alcohol also containing an asymmetric carbon atom and isomers 1 and 2 are the two isomers resulting from the asymmetric carbon atom in the alcohol portion of the ester. Both isomer 1 and isomer 2 have the same configuration in their acid portions and it is surprising that the configuration of this acid portion remains unchanged throughout the process according to the invention when the configuration of the alcohol portion changes so readily.

Separation and recovery of the isomer 2 produced may be achieved by any suitable method, for example chromatography or crystallization. Whilst the process according to the invention may be conducted in any suitable solvent, i.e., an inert material in which the starting isomer or isomers are soluble at the treatment temperature, it is preferred to employ a solvent from which isomer 2 crystallizes more readily than isomer 1. In this way it is possible to continuously remove isomer 2 from the mixture and at the same time convert isomer 1 into isomer 2 under the action of the base. The invention therefore also provides a process for the conversion of isomer 1 into isomer 2, characterized in that a solution of isomer 1 in a solvent from which isomer 2 preferentially crystallizes, alone or in

admixture with isomer 2, is treated with base at a temperature below the melting point of isomer 2, thereby providing isomer 2 in the solid phase.

In its broadest aspect the process according to the invention is carried out in a solvent which may be any inert material in which the starting material is soluble at the reaction temperature. Acidic materials, which would react with the base, are not suitable solvents. Suitable solvents include hydroxylic solvents, for example, lower alkanols containing from 1 to 4, preferably 1 or 2, carbon atoms, for example, isopropanol, butanol, ethanol, and, especially, methanol, alkanes containing from 5 to 10 carbon atoms; petroleum fractions rich in alkanes, for example, petroleum with a boiling range at atmospheric pressure of between 40°C and 65°C, between 60°C and 80°C or between 80°C and 110°C; petroleum ether, cyclohexane; mono- or dimethylcyclohexane; aromatic hydrocarbons having from 6 to 10 carbon atoms, for example benzene, toluene, *o*-, *m*- and *p*-xylene, the trimethylbenzenes and *p*-ethyl-toluene; chlorinated alkanes containing from 1 to 6, especially 1 to 4 chlorine atoms and from 1 to 4 carbon atoms, for example carbon tetra-chloride, chloroform, dichloromethane, 1,2-dichloroethane, trichloroethane and perchloroethane; chlorobenzenes, for example chlorobenzene and 1,2- or 1,3-dichlorobenzene; ethers containing from 4 to 6 carbon atoms, for example diethyl ether, diisopropyl ether, tetrahydrofuran and dioxane; nitriles containing from 2 to 6 carbon atoms, for example acetonitrile; esters having from 2 to 6 carbon atoms in each of the acid and alcohol portions, for example, ethyl acetate.

The preferred solvents, namely those from which isomer 2 crystallizes more readily than isomer 1 include hydroxylic solvents, for example, lower alkanols containing from 1 to 4, preferably 1 or 2, carbon atoms, for example isopropanol, butanol, ethanol and, especially, methanol; and alkanes containing 1 to 8 carbon atoms. For best results, the difference in solubility of isomers 1 and 2 in the selected solvent should, of course, be as high as possible.

Any base which does not itself form a stable reaction product with *alpha*-cyano-3-phenoxybenzyl *alpha*-isopropyl-4-chlorophenylacetate may be used in the process according to the invention. Preferably the base has a $pK_b$ in the reaction solvent of less than 5 and may be organic or inorganic in nature. Examples of suitable inorganic bases include hydroxides, carbonates, and cyanides of alkali and alkaline earth metals, for example, sodium cyanide, barium hydroxide, potassium hydroxide, calcium carbonate and sodium carbonate.

Suitable organic bases include alkali or alkaline earth metal salts of weak organic acids, for example, sodium acetate and magnesium formate, and organic nitrogen bases, for example alkyl, aryl or heterocyclic nitrogen bases, including mono-, di- or polyamines. Preferably, an organic nitrogen base is an amine in which any alkyl group contains from 1 to 10 carbon atoms and any aryl or aralkyl group contains from 6 to 20 carbon atoms and 1 or 2 hydrocarbyl rings. A heterocyclic amine may contain at least one ring nitrogen atom in a 5- or 6-membered heterocyclic ring optionally containing a sulphur or oxygen atom or another nitrogen atom. Suitable organic nitrogen bases include trimethylamine, triethylamine, piperidine, isoamylamine, benzylamine, diethylamine, tri-n-propylamine, tert.-butylamine, ethanolamine, tetramethylenediamine, pyridine and morpholine. Most preferred oranic nitrogen bases are secondary and, especially tertiary amines. When the amine is a tertiary amine it preferably contains three alkyl groups having 1 to 4 carbon atoms, for example, trimethylamine, tri-n-propylamine, and, especially, triethylamine.

The concentration of the base used in the process according to the invention is not critical. It may, for example, vary from 0.01 to 50 mol.% based on the amount of starting material (isomer 1 or isomers 1 + 2), and is preferably 0.05 to 20 mol.%, more preferably 0.1 to 15 mol.%. Normally about 10 mol.% is used.

The reaction may be conducted by preparing a solution of isomer 1 or a mixture of isomer 1 and isomer 2 in a suitable solvent and adding the desired amount of base to the solution. The reaction normally proceeds over a period of time of up to several days. The temperature is suitably from −50°C to +50°C, suitably −50°C to 20°C, and preferably −15°C to 5°C.

Separation and recovery of the solid product from the reaction mixture can be achieved by methods, such as filtration, centrifugation or decantation of the mother liquor. The mother liquor can then be combined with fresh quantities of isomer 1 and optionally isomer 2 and this mixture re-used in the process according to the invention.

To reduce the time required to recover the isomer 2 from the reaction mixture, it may be useful to cool the reaction mixture, separate off the precipitated crystals of isomer 2, for example by filtering, warming the mother liquor to about 50°C, and then rapidly cooling the mixture. This can be repeated several times. Seeding the reaction mixture with a small amount of crystals of relatively pure isomer 2 facilitates crystallization, but is not essential.

This preferred embodiment of the process according to the invention leads to crystalline isomer 2 in a relatively pure form. Such a compound has not previously been isolated.

In view of the fact that the presence of base may cause isomer 2, either solid or in solution, to epimerize partially to isomer 1, it may be desirable to store isomer 2 in the presence of a small quantity of an acid. For example, solid isomer 2 may be stabilized by the presence of a solid acidic clay or a solution of isomer 2 may be stabilized by the addition of a small quantity of acetic acid.

It should be noted that the process according to the invention does not require the presence of a

chiral reagent, for example a chiral catalyst. This fact facilitates the economical conversion of, for example, a mixture of isomers 1 and 2 into isomer 2. It is most surprising that the process can be performed without affecting the (+)-2-configuration of the acid portion of the ester.

The following Examples 1 and 2 illustrate the invention. Example 3 is for comparison purposes and illustrates the failure of processes analogous to those of Examples 1 and 2 when applied to chemicals closely related to those of formula (A).

EXAMPLE 1

A solution of 10.0 g of essentially equimolar amounts of isomers 1 and 2 (i.e., 10.0 g of the diastereoisomer pair, (+)-R, S-alpha-cyano-3-phenoxybenzyl (+)-S-alpha-isopropyl-4-chlorophenylacetate), and 0.1 ml of triethylamine, in 40 ml of methanol, was cooled to −10°C and seeded with a small amount of crystals of isomer 2. The mixture was kept cool for 5 days by which time more crystals had formed from the mixture in solution. The mixture was filtered and 8.2 g of solid isomer 2, m.p. 60°C, were recovered.

EXAMPLE 2

In a process analogous to that described in Example 1, isomer 2 obtained from the equimolar mixture isomers 1 and 2 using various bases and solvents as set forth in Table I below:

TABLE I

Recovery of (—)-S-alpha-cyano-3-phenoxybenzyl (+)-S-alpha-isopropyl-4-chlorophenylacetate from an equimolar diastereoisomer mixture

| Solvent | Diastereoisomer concentration g/ml | Catalyst | Time, days at −10°C | Yield, % (—)alcohol(+)acid diastereoisomer (isomer 2) |
|---|---|---|---|---|
| Methanol | 0.2 | $(C_2H_5)_3N$ | 7 | 76 |
| " | 0.25 | " | 3 | 74 |
| " | 0.25 | " | 5 | 82 |
| " | 0.25 | " | 5 | 80 |
| " | 0.33 | " | 5 | 52 |
| " | 0.25 | $Na_2CO_3$ | 14 | 70 |
| Ethanol | 0.25 | $(C_2H_5)_3N$ | 3 | 43 |
| " | 0.25 | " | 7 | 45 |
| " | 0.33 | " | 6(0°) | 60 |
| Isopropanol | 0.06 | " | 27(0°) | 37 |

EXAMPLE 3 (comparison)

The process of Example 1 was carried out using diastereoisomer mixtures of other chemicals, such as *alpha*-bromo-3-phenoxybenzyl *alpha*-isopropyl-4-chlorophenylacetate and *alpha*-ethynyl-3-phenoxybenzyl *alpha*-isopropyl-4-chlorophenylacetate, using methanol, hexane and methylene chloride as solvents, but without success.

## Claims

1. A process for the conversion of (+)-R-*alpha*-cyano-3-phenoxybenzyl (+)-S-*alpha*-isopropyl-4-chlorophenylacetate (isomer 1) into (—)-S-*alpha*-cyano-3-phenoxybenzyl (+)-S-*alpha*-isopropyl-4-chlorophenylacetate (isomer 2), characterized by treating with a base a solution of isomer 1, alone or in admixture with isomer 2, in a solvent from which isomer 2 crystallises more readily than isomer 1 and removing isomer 2 from the solution in order to permit the base to convert isomer 1 into isomer 2 up to the point at which equimolar proportions of the two isomers are present.

2. A process as claimed in claim 1, characterized in that the base is a hydroxide, carbonate or cyanide of an alkali or alkaline earth metal; an alkali or alkaline earth metal salt of a weak organic acid; or an organic nitrogen base.

3. A process as claimed in claim 2, characterized in that the base is a tertiary amine.

4. A process as claimed in claim 2, characterized in that the base is sodium carbonate or triethylamine.

5. A process as claimed in any one of claims 1 to 4, characterized in that the solvent is an alkane having from 5 to 10 carbon atoms, cyclohexane, mono- or dimethylcyclohexane, an aromatic hydrocarbon having from 6 to 10 carbon atoms, a chloroalkane having from 1 to 4 chlorine atoms and from 1 to 4 carbon atoms, mono- or dichlorobenzene, an ether having from 4 to 6 carbon atoms, a nitrile having from 2 to 6 carbon atoms, or an ester having from 2 to 6 carbons in each of the alcohol and acid portions.

6. A process as claimed in any one of claims 1 to 4, characterized in that the solvent is one from which isomer 2 crystallizes more readily than isomer 1, and the process is conducted at a temperature below the melting point of isomer 2, thereby producing solid isomer 2.

7. A process as claimed in claim 6, characterized in that the solvent is a hydroxylic solvent or an alkane having from 1 to 8 carbon atoms.

8. A process as claimed in claim 7, characterized in that the solvent is an alkanol having from 1 to 4 carbon atoms.

9. A process as claimed in any one of claims 6 to 8, characterized in that the isomer 1 is initially present together with an equimolar amount of isomer 2.

10. Crystalline (—)-S-*alpha*-cyano-3-phenoxybenzyl (+)-S-*alpha*-isopropyl-4-chlorophenylacetate.

**Revendications**

1. Un procédé pour la transformation de (+)-S-*alpha*-isopropyl-4-chlorophénylacétate de (+)-R-*alpha*-cyano-3-phénoxy-benzyle (isomère 1) en (+)-S-*alpha*-isopropyl-4-chlorophényl-acétate de (+)-S-*alpha*-isopropyl-4-chlorophénylacétate de (—)-S-alpha-cyano-3-phénoxybenzyle (isomère 2), charactérisé en ce qu'on traite par une base une solution d'isomère 1, isolément ou en mélange avec l'isomère 2, dans un solvant à partir duquel l'isomère 2 cristallise plus facilement que l'isomère 1 et on sépare l'isomère 2 de la solution de manière à permettre à la base de transformer l'isomère 1 en isomère 2 jusqu'à ce que des proportions équimolaires des deux isomères soient présentes.

2. Un procédé selon la revendication 1, caractérisé en ce que la base est un hydroxyde, carbonate ou cyanure d'un métal alcalin ou alcalino-terreux; un sel de métal alcalin ou alcalino-terreux d'un acide organique faible; ou une base azotée organique.

3. Un procédé selon la revendication 2, caractérisé en ce que la base est une amine tertiaire.

4. Un procédé selon la revendication 2, caractérisé en ce que la base est du carbonate de sodium ou de la triéthylamine.

5. Un procédé selon l'une quelconque des revendications 1 à 4, caractérisé en ce que le solvant est un alcane ayant de 5 à 10 atomes de carbone, du cyclohexane, du mono- ou diméthylcyclohexane un hydrocarbure aromatique ayant de 6 à 10 atomes de carbone, un chloro-alcane ayant de 1 à 4 atomes de chlore et de 1 à 4 atomes de carbone, du mono- dichloro-benzène, un éther ayant de 4 à 6 atomes de carbone, un nitrile ayant de 2 à 6 atomes de carbone ou un ester ayant de 2 à 6 atomes de carbone dans chacune des portions alcool et acide.

6. Un procédé selon l'une quelconque des revendications 1 à 4, caractérisé en ce que le solvant est un solvant à partir duquel l'isomère 2 cristallise plus facilement que l'isomère 1 et que le procéde est mis en oeuvre à une température au-dessous du point de fusion de l'isomère 2, de manière à produire de l'isomère 2 solide.

7. Un procédé selon la revendication 6, caractérisé en ce que le solvant est un solvant hydroxylé ou un alcane ayant de 1 à 8 atomes de carbone.

8. Un procédé selon la revendication 7, caractérisé en ce que le solvant est un alcanol ayant de 1 à 4 atomes de carbone.

9. Un procédé selon l'une quelconque des revendications 6 à 8, caractérisé en ce que l'isomère 1 est initialement présent en même temps qu'une quantité équimolaire d'isomère 2.

10. (+)-S-*alpha*-isopropyl-4-chlorophénylacétate de (—)-S-*alpha*-cyano-3-phénoxybenzyle cristallin.

**Patentansprüche**

1. Verfahren zur Umwandlung von (+)-R-$\alpha$-Cyano-3-phenoxybenzyl-(+)-S-$\alpha$-isopropyl-4-chlorphenylacetat (Isomer 1) in (—)-S-$\alpha$-Cyano-3-phenoxybenzyl-(+)-S-$\alpha$-isopropyl-4-chlorphenyl-acetat (Isomer 2), dadurch gekennzeichnet, dass eine Lösung des Isomeren 1, alleine oder im Gemisch mit Isomerem 2, in einem Lösungsmittel, aus welchem das Isomer 2 leichter kristallisiert als das Isomer 1, mit einer Base behandelt und Isomer 2 aus der Lösung entfernt wird, um zu ermöglichen, dass die Base das Isomer 1 in Isomer 2 umwandelt bis zu dem Punkt an dem äquimolare Anteilsmengen der beiden Isomeren vorhanden sind.

2. Verfahren nach Anspruch 1, dadurch gekennzeichnet, dass die Base ein Hydroxid, Carbonat

oder Cyanid eines Alkali- oder Erdalkalimetalls, ein Alkalisalz oder Erdalkalisalz einer schwachen organischen Säure oder eine organische Stickstoffbase ist.

3. Verfahren nach Anspruch 2, dadurch gekennzeichnet, dass die Base ein tertiäres Amin ist.

4. Verfahren nach Anspruch 2, dadurch gekennzeichnet, dass die Base Natriumcarbonat oder Triäthylamin ist.

5. Verfahren nach einem der Ansprüche 1 bis 4, dadurch gekennzeichnet, dass das Lösungsmittel ein Alkan mit 5 bis 10 Kohlenstoffatomen, Cyclohexan, Mono- oder Dimethylcyclohexan, ein aromatischer Kohlenwasserstoff mit 6 bis 10 Kohlenstoffatomen, ein Chloralkan mit 1 bis 4 Chloratomen sowie 1 bis 4 Kohlenstoffatomen, Mono- oder Dichlorbenzol, ein Äther mit 4 bis 6 Kohlenstoffatomen, ein Nitril mit 2 bis 6 Kohlenstoffatomen oder ein Ester mit 2 bis 6 Kohlenstoffatomen jeweils im Alkoholteil und im Säureteil ist.

6. Verfahren nach einem der Ansprüche 1 bis 4, dadurch gekennzeichnet, dass das Lösungsmittel ein solches ist, aus welchem das Isomer 2 leichter kristallisiert als das Isomer 1 und dass das Verfahren bei einer Temperatur unterhalb des Schmelzpunktes des Isomeren 2 geführt und dadurch festes Isomer 2 gewonnen wird.

7. Verfahren nach Anspruch 6, dadurch gekennzeichnet, dass das Lösungsmittel ein Hydroxylgruppen enthaltendes Lösungsmittel oder ein Alkan mit 1 bis 8 Kohlenstoffatomen ist.

8. Verfahren nach Anspruch 7, dadurch gekennzeichnet, dass das Lösungsmittel ein Alkanol mit 1 bis 4 Kohlenstoffatomen ist.

9. Verfahren nach einem der Ansprüche 6 bis 8, dadurch gekennzeichnet, dass das Isomer 1 zu Beginn zusammen mit einer äquimolaren Menge des Isomeren 2 vorhanden ist.

10. Kristallines (—)-S-$\alpha$-Cyan-3-phenoxybenzyl-(+)-S-$\alpha$-isopropyl-4-chlorphenylacetat.